# EUROPEAN PATENT SPECIFICATION

(11) **EP 2 275 401 B1**
(45) Date of publication and mention of the grant of the patent: **03.04.2013**
(21) Application number: 10013362.8
(22) Date of filing: 11.06.2003
(51) Int. Cl.: C07C 205/00, C07C 261/00, C07C 269/00, C07C 271/00

(54) **Crystalline 1-{[(alpha-isobutanoyloxyethoxy)carbonyl]aminomethyl}-1-cyclohexane acetic acid**
Kristalline 1-{[(alpha-Isobutanyloxyethoxy)carbonyl]aminomethyl}-1-cyclohexanessigsäure
Acide acétique 1-{[(alpha-isobutanoyloxyethoxy)carbonyl]aminomethyl}-1-cyclohéxane cristallin

(30) Priority: 11.06.2002 US 171485
(43) Date of publication of application: 19.01.2011
(62) Divisional of application: 03757492.8
(73) Proprietor: XenoPort, Inc., Santa Clara, CA 95051 (US)
(72) Inventor: Raillard, Stephen P., Mountain View, CA 94040 (US); Zhou, Cindy X., Palo Alto, CA 94303 (US); Yao, Fenmei, Mountain View, CA 94040 (US); Manthati, Suresh Kumar, Sunnyvale, CA 94086 (US); Xiang, Jia-Ning, Palo Alto, CA 94301 (US); Gallop, Mark A., Los Altos, CA 94022 (US)
(74) Representative: Wytenburg, Wilhelmus Johannes

(56) References cited:
- WO-A-02/100347
- WO-A1-01/90052
- US-A- 6 054 482
- POTSCHKA H; FEUERSTEIN T J; LÖSCHER W: "Gabapentin-lactam, a close analogue of the anticonvulsant gabapentin, exerts convulsant activity in amygdala kindled rats", NAUNYN-SCHMIEDEBERG'S ARCHIVES OF PHARMACOLOGY, vol. 361, 2000, pages 200-205,

## Description

### Field

Methods for synthesis of 1-(acyloxy)-alkyl carbamates are provided. More particularly, the synthesis of prodrugs (*i.e*., 1-(acyloxy)-alkyl carbamates of GABA analogs) from 1-haloalkyl carbamates of GABA analogs are described. Also described are new 1-haloalkyl carbamates of GABA analogs.

### Background

One solution to drug delivery and/or bioavailability issues in pharmaceutical development is converting known drugs to prodrugs. Typically, in a prodrug, a polar functional group (*e.g*., a carboxylic acid, an amino group, a hydroxyl group, *etc*.) is masked by a promoiety, which is labile under physiological conditions. Accordingly, prodrugs are usually transported through hydrophobic biological barriers such as membranes and typically possess superior physicochemical properties than the parent drug.

Pharmacologically effective prodrugs are non-toxic and are preferably selectively cleaved at the locus of drug action. Ideally, cleavage of the promoiety occurs rapidly and quantitatively with the formation of non-toxic by-products (*i.e*., the hydrolyzed promoiety).

The acyloxyalkoxycarbonyl functionality is an example of a promoiety that may be used to modulate the physiochemical properties of pharmaceuticals (Alexander, United States Patent No. 4,916,230; Alexander, United States Patent No. 5,733,907; Alexander et al., U.S. Patent No. 4,426,391). Typically, 1-(acyloxy)-alkyl derivatives of a pharmaceutical possess superior bioavailability, may be less irritating to topical and gastric mucosal membranes and are usually more permeable through such membranes when compared to the parent drug.

However, although 1-(acyloxy)-alkyl ester derivatives of alcohols and 1-(acyloxy)-alkyl carbamate derivatives of amines have been frequently used to mask these polar functional groups in pharmaceuticals, existing synthetic methods for preparing these desirable derivatives are inadequate. Methods disclosed in the art for synthesis of acyloxyalkyl esters and carbamates are typically multi-step routes that utilize unstable intermediates and/or toxic compounds or salts and accordingly are difficult to perform on large scale (Alexander, United States Patent No. 4,760,057; Lund, United States Patent No. 5,401,868; Alexander, United States Patent No. 4,760,057; Saari *et al*., European Patent 0416689B1).

Accordingly, there is a need for a new synthesis of 1-(acyloxy)-alkyl derivatives that proceeds rapidly and efficiently, which is amenable to scale-up and proceeds through readily accessible synthetic precursors.

WO 02/100347 A2 (XenoPort, Inc.) describes certain prodrugs of gabapentin and methods for their preparation and use. Example 13 (at page 93 therein) describes the preparation of 1-{[(a-isobutanoyloxyethoxy)carbonyl]aminomethyl}-1-cyclohexane acetic acid.

WO 01/90052 A1 (Warner-Lambert Company) describes prodrugs (including prodrugs of gabapentin) and methods for their preparation and use. Example 3 (at page 19 therein) describes 2,2-dimethyl-propionic acid 1-carboxymethylcyclohexylmethyl-carbamoyloxymethyl ester.

### Summary of the Invention

The present invention relates to crystalline 1-{[(a-isobutanoyloxyethoxy)carbonyl] aminomethyl}-1-cyclohexane acetic acid having a melting point of 63-64°C or an endotherm of 63 °C as measured by differential scanning calorimetry.

In one embodiment, the crystalline 1-{[(α-isobutanoyloxyethoxy)carbonyl]aminomethyl}-1-cyclohexane acetic acid has a melting point of 63-64°C.

In one embodiment, the crystalline 1-{[(a-isobutanoyloxyethoxy)carbonyl]aminomethyl}-1-cyclohexane acetic acid has an endotherm of 63°C as measured by differential scanning calorimetry.

In one embodiment, the crystalline 1-{[(a-isobutanoyloxyethoxy)carbonyl]aminomethyl}-1-cyclohexane acetic acid has a melting point of 63-64°C and is **obtainable by** recrystallization of 1-{[(α-isobutanoyloxyethoxy)carbonyl]aminomethyl}-1-cyclohexane acetic acid using 1 : 10 ethyl acetate : heptane.

In one embodiment, the crystalline 1-{[(α-isobutanoyloxyethoxy)carbonyl]aminomethyl}-1-cyclohexane acetic acid has an endotherm of 63°C as measured by differential scanning calorimetry, and is **obtainable by** recrystallization of 1-{[(a-isobutanoyloxyethoxy)carbonyl]aminomethyl}-1-cyclohexane acetic acid using 1 : 10 ethyl acetate : heptane.

In one embodiment, the crystalline 1-{[(a-isobutanoyloxyethoxy)carbonyl]aminomethyl}-1-cyclohexane acetic acid has a melting point of 63-64°C and is **obtainable by** recrystallization of -{[(α-isobutanoyloxyethoxy)carbonyl]aminomethyl}-1-cyclohexane acetic acid by dissolution in 1 : 10 ethyl acetate : heptane at 60°C, then slow cooling to 4°C.

In one embodiment, the crystalline 1-{[(α-isobutanoyloxyethoxy)carbonyl]aminomethyl}-1-cyclohexane acetic acid has an endotherm of 63°C as measured by differential scanning calorimetry and is **obtainable by** recrystallization of 1-{[(a-isobutanoyloxyethoxy)carbonyl]aminomethyl}-1-cyclohexane acetic acid by dissolution in 1 : 10 ethyl acetate : heptane at 60°C, then slow cooling to 4°C.

In one embodiment, the crystalline 1-{[(a-isobutanoyloxyethoxy)carbonyl]aminomethyl}-1-cyclohexane acetic acid has a melting point of 63-64°C and is **obtainable by** recrystallization of 1-{[(α-isobutanoyloxyethoxy)carbonyl]aminomethyl}-1-cyclohexane acetic acid by dissolution in heptane at 70°C, then slow cooling to room temperature.

### Detailed Description

### Definitions

"Compounds" refers to compounds encompassed by structural formulae **(I) - (VIII)** disclosed herein and includes any specific compounds within these formulae whose structure is disclosed herein. Compounds may be identified either by their chemical structure and/or chemical name. When the chemical structure and chemical name conflict, the chemical structure is determinative of the identity of the compound. The compounds described herein may contain one or more chiral centers and/or double bonds and therefore, may exist as stereoisomers, such as double-bond isomers (*i.e*., geometric isomers), enantiomers or diastereomers. Accordingly, the chemical structures depicted herein encompass all possible enantiomers and stereoisomers of the illustrated compounds including the stereoisomerically pure form (*e.g*., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The compounds may also exist in several tautomeric forms including the enol form, the keto form and mixtures thereof. Accordingly, the chemical structures depicted herein encompass all possible tautomeric forms of the illustrated compounds. The compounds described also include isotopically labeled compounds where one or more atoms have an atomic mass different from the atomic mass conventionally found in nature. Examples of isotopes that may be incorporated into the compounds described herein include, but are not limited to, ²H, ³H, ¹³C, ¹⁴C, ¹⁵N, ¹⁸O, ¹⁷O, etc. Compounds may exist in unsolvated forms as well as solvated forms, including hydrated forms and as N-oxides. In general, compounds may be hydrated, solvated or N-oxides. Further, it should be understood, when partial structures of the compounds are illustrated, that brackets indicate the point of attachment of the partial structure to the rest of the molecule.

"1-Acyloxy-Alkyl Carbamate" refers to an N-1-acyloxy-alkoxycarbonyl derivative of a primary or secondary amine as encompassed by structural formulae **(I)**, **(V)** and **(VI)** disclosed herein.

"Alkyl" by itself or as part of another substituent refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl (allyl), cycloprop-1-en-1-yl; cycloprop-2-en-1-yl, prop-1-yn-1-yl, prop-2-yn-1-yl, *etc*.; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc*.; and the like.

The term "alkyl" is specifically intended to include groups having any degree or level of saturation, i.e., groups having exclusively single carbon-carbon bonds, groups having one or more double carbon-carbon bonds, groups having one or more triple carbon-carbon bonds and groups having mixtures of single, double and triple carbon-carbon bonds. Where a specific level of saturation is intended, the expressions "alkanyl," "alkynyl," and "alkynyl" are used. Preferably, an alkyl group comprises from 1 to 20 carbon atoms, more preferably, from 1 to 10 carbon atoms, most preferably, from 1 to 6 carbon atoms.

"Alkanyl" by itself or as part of another substituent refers to a saturated branched, straight-chain or cyclic alkyl radical derived by the removal of one hydrogen atom from a single carbon atom of a parent alkane. Typical alkanyl groups include, but are not limited. to, methanyl; ethanyl; propanyls such as propan-1-yl, propan-2-yl (isopropyl), cyclopropan-1-yl, *etc*.; butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc*.; and the like.

"Alkenyl" by itself or as part of another substituent refers to an unsaturated branched, straight-chain or cyclic alkyl radical having at least one carbon-carbon double bond derived by the removal of one hydrogen atom from a single carbon atom of a parent alkene. The group may be in either the cis or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc.;* and the like.

"Alkynyl" by itself or as part of another substituent refers to an unsaturated branched, straight-chain or cyclic alkyl radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, *etc*.; butynyls such as but-1-yn-1-yl, but-1-yn-3-yl, but-3-yn-1-yl, *etc*.; and the like.

"Acyl" by itself or as part of another substituent refers to a radical -C(O)R³⁰, where R³⁰ is hydrogen, alkyl, cycloalkyl, cycloheteroalkyl, aryl, arylalkyl, heteroalkyl, heteroaryl, heteroarylalkyl as defined herein. Representative examples include, but are not limited to formyl, acetyl, cyclohexylcarbonyl, cyclohexylmethylcarbonyl, benzoyl, benzylcarbonyl and the like.

"Alkoxy" by itself or as part of another substituent refers to a radical -OR³¹ where R³¹ represents an alkyl or cycloalkyl group as defined herein. Representative examples include, but are not limited to, methoxy, ethoxy, propoxy, butoxy, cyclohexyloxy and the like.

"Alkoxycarbonyl" by itself or as part of another substituent refers to a radical -OR³² where R³² represents an alkyl or cycloalkyl group as defined herein. Representative examples include, but are not limited to, methoxycarbonyl, ethoxycarbonyl, propoxycarbonyl, butoxycarbonyl, cyclohexyloxycarbonyl and the like.

"Aryl" by itself or as part of another substituent refers to a monovalent aromatic hydrocarbon radical derived by the removal of one hydrogen atom from a single carbon atom of a parent aromatic ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. Preferably, an aryl group comprises from 6 to 20 carbon atoms, more preferably, from 6 to 12 carbon atoms.

"Arylalkyl" by itself or as part of another substituent refers to an acyclic alkyl radical in which one of the hydrogen atoms bonded to a carbon atom, typically a terminal or sp³ carbon atom, is replaced with an aryl group. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylalkenyl and/or arylalkynyl is used. Preferably, an arylalkyl group is (C₆-C₃₀) arylalkyl, *e.g*., the alkanyl, alkenyl or alkynyl moiety of the arylalkyl group is (C₁-C₁₀) and the aryl moiety is (C₆-C₂₀), more preferably, an arylalkyl group is (C₆-C₂₀) arylalkyl, *e.g*., the alkanyl, alkenyl or alkynyl moiety of the arylalkyl group is (C₁-C₈) and the aryl moiety is (C₆-C₁₂)·

"Aryldiakylsilyl" by itself or as part of another substituent refers to the radical - SiR³³R³⁴R³⁵ where one of R³³, R³⁴ or R³⁵ is aryl as defined herein and the other two of R³³, R³⁴ or R³⁵ are alkyl as defined herein.

"Bridged cycloalkyl" by itself or as part of another substituent refers to a radical 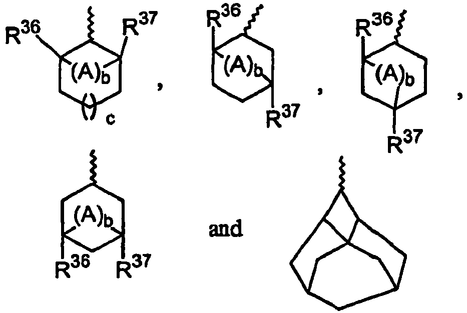 selected from the group consisting of
wherein:
A is (CR³⁸R³⁹)_{b};
R³⁸ and R³⁹ are independently selected from the group consisting of hydrogen and methyl;
R³⁶ and R³⁷ are independently selected from the group consisting of hydrogen and methyl;
b is an integer from 1 to 4; and
c is an integer from 0 to 2.

"Carbamoyl" by itself or as part of another substituent refers to the radical -C(O)NR⁴⁰R⁴¹ where R⁴⁰ and R⁴¹ are independently hydrogen, alkyl, cycloalkyl or aryl as defined herein.

"Cycloalkyl" by itself or as part of another substituent refers to a saturated or unsaturated cyclic alkyl radical. Where a specific level of saturation is intended, the nomenclature "cycloalkanyl" or "cycloalkenyl" is used. Typical cycloalkyl groups include, but are not limited to, groups derived from cyclopropane, cyclobutane, cyclopentane, cyclohexane and the like. Preferably, the cycloalkyl group is (C₃-C₁₀) cycloalkyl, more preferably (C₃-C₇) cycloalkyl.

"Cycloheteroalkyl" by itself or as part of another substituent refers to a saturated or unsaturated cyclic alkyl radical in which one or more carbon atoms (and any associated hydrogen atoms) are independently replaced with the same or different heteroatom. Typical heteroatoms to replace the carbon atom(s) include, but are not limited to, N, P, O, S, Si, *etc.* Where a specific level of saturation is intended, the nomenclature "cycloheteroalkanyl" or "cycloheteroalkenyl" is used. Typical cycloheteroalkyl groups include, but are not limited to, groups derived from epoxides, azirines, thiiranes, imidazolidine, morpholine, piperazine, piperidine, pyrazolidine, pyrrolidine, quinuclidine, and the like.

"GABA analog" refers to a compound, unless specified otherwise, as having the following structure: 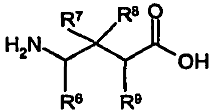 wherein:
R⁶ and R⁹ are independently selected from the group consisting of hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroaryl, substituted heteroaryl, heteroarylalkyl and substituted heteroarylalkyl;
R⁷ and R⁸ are independently selected from the group consisting of hydrogen, alkyl, substituted alkyl, acyl, substituted acyl, arylalkyl, substituted arylalkyl, cycloalkyl, substituted cycloalkyl, cycloheteroalkyl; substituted cycloheteroalkyl, heteroarylalkyl and substituted heteroarylalkyl, or optionally, R⁷ and R⁸ together with the carbon atom to which they are attached form a cycloalkyl, substituted cycloalkyl, cycloheteroalkyl, substituted cycloheteroalkyl or bridged cycloalkyl ring.

"1-Haloalkyl Carbamate" refers to an N-1-haloalkoxycarbonyl derivative of a primary or secondary amine as encompassed by structural formulae (**II**), (**VII**) and (**VIII**) disclosed herein.

"Heteroalkyl, Heteroalkanyl, Heteroalkenyl and Heteroalkynyl" by themselves or as part of another substituent refer to alkyl, alkanyl, alkenyl and alkynyl groups, respectively, in which one or more of the carbon atoms (and any associated hydrogen atoms) are independently replaced with the same or different heteroatomic groups. Typical heteroatomic groups which can be included in these groups include, but are not limited to, -O-, -S-, -O-O-, -S-S-, -O-S-, -NR⁴²R⁴³, -=N-N=- -N=N-, -N=N-NR⁴⁴R⁴⁵, -PR⁴⁶-, -P(O)₂-, -POR⁴⁷-, -O-P(O)₂-, -SO-, -SO₂-, -SnR⁴⁸R⁴⁹- and the like, where R⁴², R⁴³, R⁴⁴, R⁴⁵, R⁴⁶, R⁴⁷, R⁴⁸ and R⁴⁹ are independently hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl.

"Heteroaryl" by itself or as part of another substituent refers to a monovalent heteroaromatic radical derived by the removal of one hydrogen atom from a single atom of a parent heteroaromatic ring system. Typical heteroaryl groups include, but are not limited to, groups derived from acridine, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. Preferably, the heteroaryl group is from 5-20 membered heteroaryl, more preferably from 5-10 membered heteroaryl. Preferred heteroaryl groups are those derived from thiophene, pyrrole, benzothiophene, benzofuran, indole, pyridine, quinoline, imidazole, oxazole and pyrazine

"Heteroarylalkyl" by itself or as part of another substituent refers to an acyclic alkyl radical in which one of the hydrogen atoms bonded to a carbon atom, typically a terminal or *sp*³ carbon atom, is replaced with a heteroaryl group. Where specific alkyl moieties are intended, the nomenclature heteroarylalkanyl, heteroarylalkenyl and/or heterorylalkynyl is used. In preferred embodiments, the heteroarylalkyl group is a 6-30 membered heteroarylalkyl, *e.g*., the alkanyl, alkenyl or alkynyl moiety of the heteroarylalkyl is 1-10 membered and the heteroaryl moiety is a 5-20-membered heteroaryl, more preferably, 6-20 membered heteroarylalkyl, *e.g*., the alkanyl, alkenyl or alkynyl moiety of the heteroarylalkyl is 1-8 membered and the heteroaryl moiety is a 5-12-membered heteroaryl.

"Parent Aromatic Ring System" refers to an unsaturated cyclic or polycyclic ring system having a conjugated π electron system. Specifically included within the definition of "parent aromatic ring system" are fused ring systems in which one or more of the rings are aromatic and one or more of the rings are saturated or unsaturated, such as, for example, fluorene, indane, indene, phenalene, *etc*. Typical parent aromatic ring systems include, but are not limited to, aceanthrylene, acenaphthylene, acephenanthrylene, anthracene, azulene, benzene, chrysene, coronene, fluoranthene, fluorene, hexacene, hexaphene, hexalene, *as*-indacene, *s*-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like.

"Parent Heteroaromatic Ring System" refers to a parent aromatic ring system in which one or more carbon atoms (and any associated hydrogen atoms) are independently replaced with the same or different heteroatom. Typical heteroatoms to replace the carbon atoms include, but are not limited to, N, P, O, S, Si, *etc*. Specifically included within the definition of "parent heteroaromatic ring systems" are fused ring systems in which one or more of the rings are aromatic and one or more of the rings are saturated or unsaturated, such as, for example, arsindole, benzodioxan, benzofuran, chromane, chromene, indole, indoline, xanthene, *etc*. Typical parent heteroaromatic ring systems include, but are not limited to, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like.

"Pharmaceutically acceptable salt" refers to a salt of a compound, which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid; propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced by a metal ion, *e.g*., an alkali metal ion, an alkaline earth ion, or an aluminum ion; or coordinates with an organic base such as ethanolamine, diethanolamine, triethanolamine, N-methylglucamine and the like.

"Prodrug" refers to a derivative of a drug molecule that requires a transformation within the body to release the active drug. Prodrugs are frequently, although not necessarily, pharmacologically inactive until converted to the parent drug. A hydroxyl containing drug may be converted to, for example, to a sulfonate, ester or carbonate prodrug, which may be hydrolyzed *in vivo* to provide the hydroxyl compound. An amino containing drug may be converted, for example, to a carbamate, amide, enamine, imine, N-phosphonyl, N-phosphoryl or N-sulfenyl prodrug, which may be hydrolyzed *in vivo* to provide the amino compound. A carboxylic acid drug may be converted to an ester (including silyl esters and thioesters), amide or hydrazide prodrug, which be hydrolyzed *in vivo* to provide the carboxylic acid compound. Prodrugs for drugs which have functional groups different than those listed above are well known to the skilled artisan.

"Promoiety" refers to a form of protecting group that when used to mask a functional group within a drug molecule converts the drug into a prodrug. Typically, the promoiety will be attached to the drug *via* bond(s) that are cleaved by enzymatic or non-enzymatic means *in vivo.*

"Protecting group" refers to a grouping of atoms that when attached to a reactive functional group in a molecule masks, reduces or prevents reactivity of the functional group. Examples of protecting groups can be found in Green et al., "Protective Groups in Organic Chemistry", (Wiley, 2nd ed. 1991) and Harrison et al., "Compendium of Synthetic Organic Methods", Vols. 1-8 (John Wiley and Sons, 1971-1996). Representative amino protecting groups include, but are not limited to, formyl, acetyl, trifluoroacetyl, benzyl, benzyloxycarbonyl ("CBZ"), *tert*-butoxycarbonyl ("Boc"), trimethylsilyl ("TMS"), 2-trimethylsilyl-ethanesulfonyl ("SES"), trityl and substituted trityl groups, allyloxycarbonyl, 9-fluorenylmethyloxycarbonyl ("FMOC"), nitro-veratryloxycarbonyl ("NVOC") and the like. Representative hydroxy protecting groups include, but are not limited to, those where the hydroxy group is either acylated or alkylated such as benzyl, and trityl ethers as well as alkyl ethers, tetrahydropyranyl ethers, trialkylsilyl ethers and allyl ethers.

"Substituted" refers to a group in which one or more hydrogen atoms are independently replaced with the same or different substituent(s). Typical substituents include, but are not limited to, -M, -R⁶⁰, -O⁻, =O, -OR⁶⁰, -SR⁶⁰, -S⁻, =S, NR⁶⁰R⁶¹, =NR⁶⁰, -CF₃, -CN, -OCN, -SCN, -NO, -NO₂, =N₂, -N₃, -S(O)₂O⁻, -S(O)₂OH, -S(O)₂R⁶⁰, -OS(O₂)O⁻, -OS(O)₂R⁶⁰, -P(O)(O⁻)₂, -P(O)(OR⁶⁰)(O⁻), -OP(O)(OR⁶⁰)(OR⁶¹), -C(O)R⁶⁰, -C(S)R⁶⁰, -C(O)OR⁶⁰, -C(O)NR⁶⁰R⁶¹, C(O)O⁻, -C(S)OR⁶⁰, -NR⁶²C(O)NR⁶⁰R⁶¹, -NR⁶²C(S)NR⁶⁰R⁶¹, -NR⁶²C(NR⁶³)NR⁶⁰R⁶¹ and -C(NR⁶²)NR⁶⁰R⁶¹ where M is independently a halogen; R⁶⁰, R⁶¹, R⁶² and R⁶³ are independently hydrogen, alkyl, substituted alkyl, alkoxy, substituted alkoxy, cycloalkyl, substituted cycloalkyl, cycloheteroalkyl, substituted cycloheteroalkyl, aryl, substituted aryl, heteroaryl or substituted heteroaryl, or optionally R⁶⁰ and R⁶¹ together with the nitrogen atom to which they are bonded form a cycloheteroalkyl or substituted cycloheteroalkyl ring; and R⁶⁴ and R⁶⁵ are independently hydrogen, alkyl, substituted alkyl, aryl, cycloalkyl, substituted cycloalkyl, cycloheteroalkyl, substituted cycloheteroalkyl, aryl, substituted aryl, heteroaryl or substituted heteroaryl, or optionally R⁶⁴ and R⁶⁵ together with the nitrogen atom to which they are bonded form a cycloheteroalkyl or substituted cycloheteroalkyl ring. Preferably, substituents include -M, -R⁶⁰, =O, -OR⁶⁰, -SR⁶⁰, -S⁻, =S, -NR⁶⁰R⁶¹, =NR⁶⁰, -CF₃, -CN, -OCN, -SCN, -NO, -NO₂, =N₂, -N₃, -S(O)₂R⁶⁰, -OS(O₂)O⁻, -OS(O)₂R⁶⁰, -P(O)(O⁻)₂, -P(O)(OR⁶⁰)(O⁻), -OP(O)(OR⁶⁰)(OR⁶¹), -C(O)R⁶⁰, -C(S)R⁶⁰, -C(O)OR⁶⁰, -C(O)NR⁶⁰R⁶¹,-C(O)O⁻, -NR⁶²C(O)NR⁶⁰R⁶¹, more preferably, -M, -R⁶⁰, =O, -OR⁶⁰, -SR⁶⁰, -NR⁶⁰R⁶¹, -CF₃, -CN, -NO₂, -S(O)₂R⁶⁰, -P(O)(OR⁶⁰)(O⁻), -OP(O)(OR⁶⁰)(OR⁶¹), -C(O)R⁶⁰, -C(O)OR⁶⁰, -C(O)NR⁶⁰R⁶¹,-C(O)O-, most preferably, -M, -R⁶⁰, =O, -OR⁶⁰, -SR⁶⁰, -NR⁶⁰R⁶¹, -CF₃, -CN, -NO₂, -S(O)₂R⁶⁰, -OP(O)(OR⁶⁰)(OR⁶¹), -C(O)R⁶⁰, -C(O)OR⁶⁰ ,-C(O)O⁻, where R⁶⁰, R⁶¹ and R⁶² are as defined above.

"Trialkylsilyl" by itself or as part of another substituent refers to a radical - SiR⁵⁰R⁵¹R⁵² where R⁵⁰, R⁵¹ and R⁵² are alkyl as defined herein.

### Method of Synthesis of 1-(Acyloxy)-Alkyl GABA Analogs

Methods of synthesis of 1-(acyloxy)-alkyl carbamates of GABA analogs from 1-haloalkyl carbamates of GABA analogs are disclosed. Preferably, 1-(acyloxy)-alkyl carbamates of GABA analogs are synthesized by reaction of a 1-haloalkyl carbamate of a GABA analog with a carboxylic acid in the presence of either a metal salt or an organic base. In one embodiment, the carboxylic acid also serves a solvent for the reaction.

In a first approach, a compound of Formula (**I**) is synthesized by a method comprising 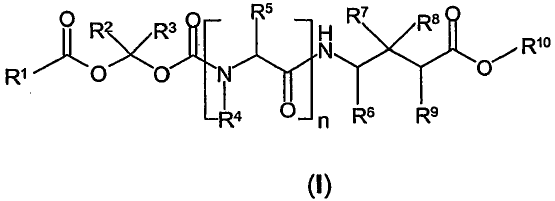 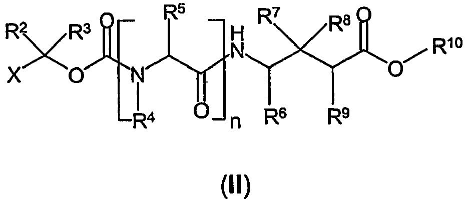 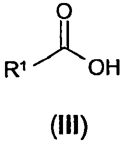 contacting a compound of Formula (II), a compound of Formula (**III**) and at least one equivalent of a metal salt wherein:
X is F, Cl, Br or I;
n is 0 or 1;
R¹ is acyl, substituted acyl, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R² and R³ are independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or optionally, R² and R³ together with the atom to which they are bonded form a cycloalkyl, substituted cycloalkyl, cycloheteroalkyl or substituted cycloheteroalkyl ring;
R⁴ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R⁵ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or optionally, R⁴ and R⁵ together with the atoms to which they are attached form a cycloheteroalkyl or substituted cycloheteroalkyl ring;
R⁶ and R⁹ are independently selected from the group consisting of hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroaryl, substituted heteroaryl, heteroarylalkyl and substituted heteroarylalkyl;
R⁷ and R⁸ are independently selected from the group consisting of hydrogen, alkyl, substituted alkyl, acyl, substituted acyl, arylalkyl, substituted arylalkyl, cycloalkyl, substituted cycloalkyl, cycloheteroalkyl, substituted cycloheteroalkyl, heteroarylalkyl and substituted heteroarylalkyl, or optionally, R⁷and R⁸ together with the carbon atom to which they are attached form a cycloalkyl, substituted cycloalkyl, cycloheteroalkyl, substituted cycloheteroalkyl or bridged cycloalkyl ring; and
R¹⁰ is hydrogen, alkyl, substituted alkyl, aryl, substituted aryl, arylalkyl, substituted arylalkyl, aryldialkylsilyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl, substituted heteroarylalkyl or trialkylsilyl.

In a second approach, a compound of Formula (**I**) is synthesized by a method comprising contacting a compound of Formula (**II**), a compound of Formula (**III**) and at least one equivalent of an organic base, wherein X, n, R¹, R², R³, R⁴, R⁵, R⁶, R⁷, R⁸, R⁹ and R¹⁰ are as defined, *supra.*

In one embodiment, a compound of Formulae (**I**) or (**II**) is derived from a GABA analog of Formula (**IV**): 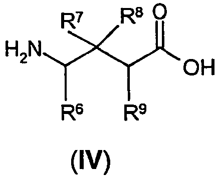
wherein the GABA analog of Formula (**IV**) is
1-Aminomethyl-1-cyclohexane acetic acid (i.e. gabapentin).

In another embodiment, the compound of Formula (**I**) is a compound of Formulae (**V**) or (**VI**):

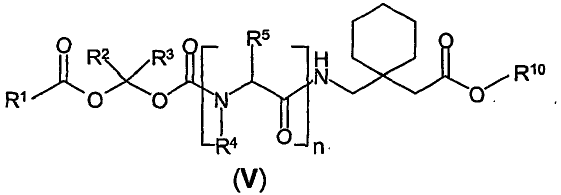

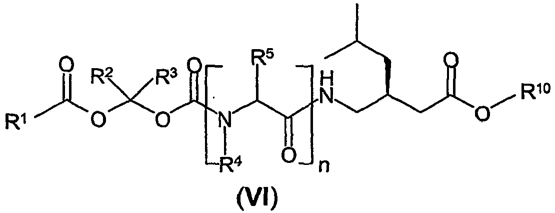

The skilled artisan will appreciate that the embodiments, *infra*, refer to compounds of Formulae (**V**) and (**VI**)

In one embodiment, n is 0.

In another embodiment, R¹ is methyl, ethyl, propyl, isopropyl, butyl, isobutyl, *sec*-butyl, *tert*-butyl, pentyl, isopentyl, *sec*-pentyl, neopentyl, 1,1,-dimethoxyethyl, 1,1-diethoxyethyl, cyclobutyl, cyclopentyl, cyclohexyl, phenyl, 4-methoxyphenyl, benzyl, phenethyl, styryl or 3-pyridyl, R² is methyl and R³ is hydrogen.

In another embodiment, R² is hydrogen, methyl, ethyl, *n*-propyl, isopropyl, *n*-butyl, isobutyl, *sec*-butyl or *tert*-butyl and R³ is hydrogen.

In one embodiment, R¹ is methyl, ethyl, *n*-propyl, isopropyl, *n*-butyl, isobutyl, *sec*-butyl, *tert*-butyl,1,1-dimethoxyethyl, 1,1-diethoxyethyl, cyclobutyl, cyclopentyl or cyclohexyl, and R¹⁰ is hydrogen, allyl, benzyl or trimethylsilyl.

In one embodiment, R¹ is methyl, ethyl, *n*-propyl, isopropyl, *n*-butyl, isobutyl, *sec*-butyl, *tert*-butyl, 1,1-dimethoxyethyl, 1,1-diethoxyethyl, cyclobutyl, cyclopentyl or cyclohexyl, R¹⁰ is hydrogen, allyl, benzyl or trimethylsilyl, R² is hydrogen, methyl, ethyl, isopropyl, *n*-butyl, isobutyl, *sec*-butyl or *tert*-butyl, and R³ is hydrogen. In another embodiment, R¹ is ethyl or isopropyl, R¹⁰ is allyl, benzyl or trimethylsilyl, R² is methyl, *n*-propyl or isopropyl, and R³ is hydrogen. In still another embodiment, R¹ is isopropyl, R¹⁰ is benzyl, R² is methyl, and R³ is hydrogen. In still another embodiment, R¹ is isopropyl, R¹⁰ is allyl, R² is methyl, and R³ is hydrogen.

In one embodiment, R¹ is ethyl or isopropyl, R¹⁰ is allyl, benzyl or trimethylsilyl, R² is methyl, *n*-propyl or isopropyl, R³ is hydrogen and X is chloro. In another embodiment, R¹ is isopropyl, R¹⁰ is benzyl, R² is methyl, R³ is hydrogen and X is chloro. In still another embodiment, R¹ is isopropyl, R¹⁰ is allyl, R² is methyl, R³ is hydrogen and X is chloro.

In one embodiment, X is bromo or chloro. In still another embodiment, X is chloro, bromo or iodo, R¹⁰ is hydrogen, allyl, benzyl or trimethylsilyl, R² is methyl and R³ is hydrogen.

In still another embodiment, X is chloro, bromo or iodo, R¹⁰ is hydrogen, allyl, benzyl or trimethylsilyl and R² and R³ together with the atom to which they are attached form a cyclohexyl ring. In still another embodiment, X is chloro, R¹⁰ is hydrogen, allyl, benzyl or trimethylsilyl, R² is hydrogen, methyl, ethyl, *n*-propyl, isopropyl, *n*-butyl, isobutyl, *sec*-butyl or *tert*-butyl and R³ is hydrogen. In still another embodiment, X is chloro, R¹⁰ is hydrogen, allyl, benzyl or trimethylsilyl, R² is methyl, *n*-propyl or isopropyl, and R³ is hydrogen. In still another embodiment, X is chloro, R¹⁰ is allyl, R² is methyl, and R³ is hydrogen. In still another embodiment, X is chloro, R¹⁰ is benzyl, R² is methyl, and R³ is hydrogen. In still another embodiment, X is chloro, R¹⁰ is trimethylsilyl, R² is methyl, and R³ is hydrogen.

Those of skill in the art will appreciate that the following embodiments, *infra*, refer to compounds of Formulae (**I**), (**II**) and (**III**). In one embodiment, the ratio of the compound of Formula (**II**) to the compound of Formula (**III**) is between about 1:1 and 1:20. In another embodiment, the ratio of the compound of Formula (**II**) to the compound of Formula (**III**) is between about 1:1 and 1:5. In still another embodiment, the ratio of the compound of Formula (**II**) to the compound of Formula (**III**) is about 1:1.

In one embodiment, the compounds of Formulae (**II**) and (**III**) and the metal salt are contacted with a solvent. In another embodiment, the ratio of the compound of Formula (**II**) to the compound of Formula (**III**) is between about 1:1 and 1:20. In still another embodiment, the ratio of the compound of Formula (**II**) to the compound of Formula (**III**) is between about 1:1 and 1:5. In still another embodiment, the ratio of the compound of Formula (**II**) to the compound of Formula (**III**) is about 1:1. In one embodiment, the solvent is dichloromethane, dichloroethane, chloroform, toluene, dimethylformamide, dimethylacetamide, N-methylpyrrolidinone, dimethyl sulfoxide, pyridine, ethyl acetate, acetonitrile, acetone, 2-butanone, methyl *ter*t-butyl ether, methanol, ethanol, isopropanol, *ter*t-butanol, water, hexamethylphosphoramide or combinations thereof. In another embodiment, the metal is Ag, Hg, Na, K, Li, Cs, Ca, Mg or Zn.

In one embodiment, the compounds of Formulae (**II**) and (**III**) and the organic base are contacted with a solvent. In another embodiment, the ratio of the compound of Formula (**II**) to the compound of Formula (**III**) is between about 1:1 and 1:20. In still another embodiment, the ratio of the compound of Formula (**II**) to the compound of Formula (**III**) is between about 1:15 and 1:20. In still another embodiment, the ratio of the compound of Formula (**II**) to the compound of Formula (**III**) is about 1:10. In still another embodiment, the ratio of the compound of Formula (**II**) to the compound of Formula (**III**) is between about 1:1 and 1:5. In still another embodiment, the ratio of the compound of Formula (**II**) to the compound of Formula (**III**) is about 1:1. In one embodiment, the solvent is dichloromethane, dichloroethane, chloroform, toluene, dimethylformamide, dimethylacetamide, N-methylpyrrolidinone, dimethyl sulfoxide, pyridine, ethyl acetate, acetonitrile, acetone, 2-butanone, methyl *tert*-butyl ether, methanol, ethanol, isopropanol, *tert*-butanol, water, hexamethylphosphoramide or combinations thereof. In another embodiment, the organic base is triethylamine, tributylamine, diisopropylethylamine, dimethylisopropylamine, N-methylinorpholine, N-methylpyrrolidine, N-methylpiperidine, pyridine, 2-methylpyridine, 2,6-dimethylpyridine, 4-dimethylaminopyridine, 1, 4-diazabicyclo[2.2.2]octane, 1, 8-diazabicyclo[5.4.0]undec-7-ene, 1, 5-diazabicyclo[4.3.0]undec-7-ene or combinations thereof.

In one embodiment, the compound of Formula (**III**) is a liquid under the conditions of said contacting, the compound of Formula (**III**) further serving as a solvent for the reaction with the compound of Formula (**II**). In another embodiment, the compound of Formula (**III**) is acetic acid, methoxyacetic acid, ethoxyacetic acid, propionic acid, butyric acid, isobutyric acid, pivalic acid, valeric acid, isovaleric acid, 2-methylbutyric acid, cyclobutanecarboxylic acid, cyclopentanecarboxylic acid or cyclohexanecarboxylic acid. In still another embodiment, the compound of Formula (**III**) is isobutyric acid.

In one embodiment, the compound of Formula (**II**), the compound of Formula (**III**) and the metal salt are contacted at a temperature between about -25 °C and about 120 °C. In another embodiment, the temperature is between about 0 °C and about 25 °C.

In one embodiment, the compound of Formula (**II**), the compound of Formula (**III**) and the organic base are contacted at a temperature between about -25 °C and about 120 °C. In another embodiment, the temperature is between about 0 °C and about 25 °C.

In one embodiment, the compound of Formula (**II**), the compound of Formula (**III**) and the organic base are contacted with a catalytic amount of an iodide salt. In still another embodiment, the iodide salt is sodium iodide, potassium iodide, tetramethylammonium iodide, tetraethylammonium iodide or tetrabutylammonium iodide.

### 1-Haloalkyl Carbamates of GABA Analogs

Also disclosed herein are 1-haloalkyl carbamates of GABA analogs of Formula (II): 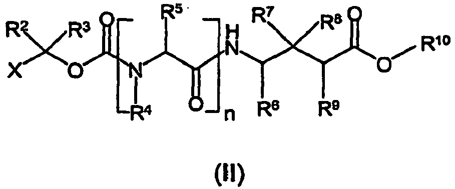 wherein:
X is a halogen;
n is 0 or 1;
R² and R³ are independently hydrogen, alkyl, substituted alkyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, heteroalkyl, substituted heteroalkyl, heteroaryl, substituted heteroaryl, heteroarylalkyl or substituted heteroarylalkyl, or optionally, R² and R³ together with the atom to which they are bonded form a cycloalkyl, substituted cycloalkyl, cycloheteroalkyl or substituted cycloheteroalkyl ring;
R⁴ is hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl;
R⁵ is hydrogen, alkyl, substituted alkyl, alkoxy, substituted alkoxy, acyl, substituted acyl, alkoxycarbonyl, substituted alkoxycarbonyl, aryl, substituted aryl, arylalkyl, substituted arylalkyl, carbamoyl, substituted carbamoyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl, or optionally, R⁴ and R⁵ together with the atoms to which they are attached form a cycloheteroalkyl or substituted cycloheteroalkyl ring;
R⁶ and R⁹ are independently selected from the group consisting of hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroaryl, substituted heteroaryl, heteroarylalkyl and substituted heteroarylalkyl;
R⁷ and R⁸ are independently selected from the group consisting of hydrogen, alkyl, substituted alkyl, acyl, substituted acyl, arylalkyl, substituted arylalkyl, cycloalkyl, substituted cycloalkyl, cycloheteroalkyl, substituted cycloheteroalkyl, heteroarylalkyl and substituted heteroarylalkyl, or optionally, R⁷ and R⁸ together with the carbon atom to which they are attached form a cycloalkyl, substituted cycloalkyl, cycloheteroalkyl, substituted cycloheteroalkyl or bridged cycloalkyl ring; and
R¹⁰ is hydrogen, alkyl, substituted alkyl, aryl, substituted aryl, arylalkyl, substituted arylalkyl, aryldialkylsilyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl, substituted heteroarylalkyl or trialkylsilyl.

### Synthesis of 1-Haloalkyl Carbamates and Conversion to 1-Acylox₋Alkyl Carbamates

The compounds and methods described herein may be obtained and/or practiced according to the synthetic methods illustrated in Schemes 1-5. Numerous methods have been described in the art for the synthesis of GABA analogs (*i.e*., compounds of formula *(1), infra,* where n = 0; see, *e.g*., Satzinger *et al.,* United States Patent No. 4,024,175; Silverman *et al.,* United States Patent No. 5,563,175; Horwell *et al.,* United States Patent No. 6,020,370; Silverman *et al.,* United States Patent No. 6,028,214; Horwell *et al.,* United States Patent No. 6,103,932; Silverman *et al.,* United States Patent No. 6,117,906; Silverman, International Publication No. WO 92/09560; Silverman et al., International Publication No. WO 93/23383; Horwell et al., International Publication No. WO 97/29101, Horwell et al., International Publication No. WO 97/33858; Horwell et al., International Publication No. WO 97/33859; Bryans et al., International Publication No. WO 98/17627; Guglietta et al., International Publication No. WO 99/08671; Bryans et al., International Publication No. WO 99/21824; Bryans et al., International Publication No. WO 99/31057; Belliotti et al., International Publication No. WO 99/31074; Bryans et al., International Publication No. WO 99/31075; Bryans et al., International Publication No. WO 99/61424; Bryans et al., International Publication No. WO 00/15611; Bryans, International Publication No. WO 00/31020; and Bryans et al., International Publication No. WO 00/50027). Other methods are known in the art for synthesizing GABA analogs, which are readily accessible to the skilled artisan. Methods for synthesis of amino acid derivatives of GABA analogs (*i.e*., compounds of formula (**1**), *infra*, where n = 1) are disclosed in Gallop *et al.,* International Publication No. WO 02/100347).

Accordingly, starting materials useful for preparing compounds and intermediates thereof, and/or practicing methods (described herein are commercially available or can be prepared by well-known synthetic methods. Other methods for synthesis of the prodrugs described herein are either described in the art or will be readily apparent to the skilled artisan in view of the references provided above and may be used to synthesize the compounds described herein. Accordingly, the methods presented in the Schemes herein are illustrative rather than comprehensive.

Intermediate (**4**) useful in the preparation of 1-haloallcyl carbamates of Formula (**II**) may be generated according to reactions detailed in Scheme 1.

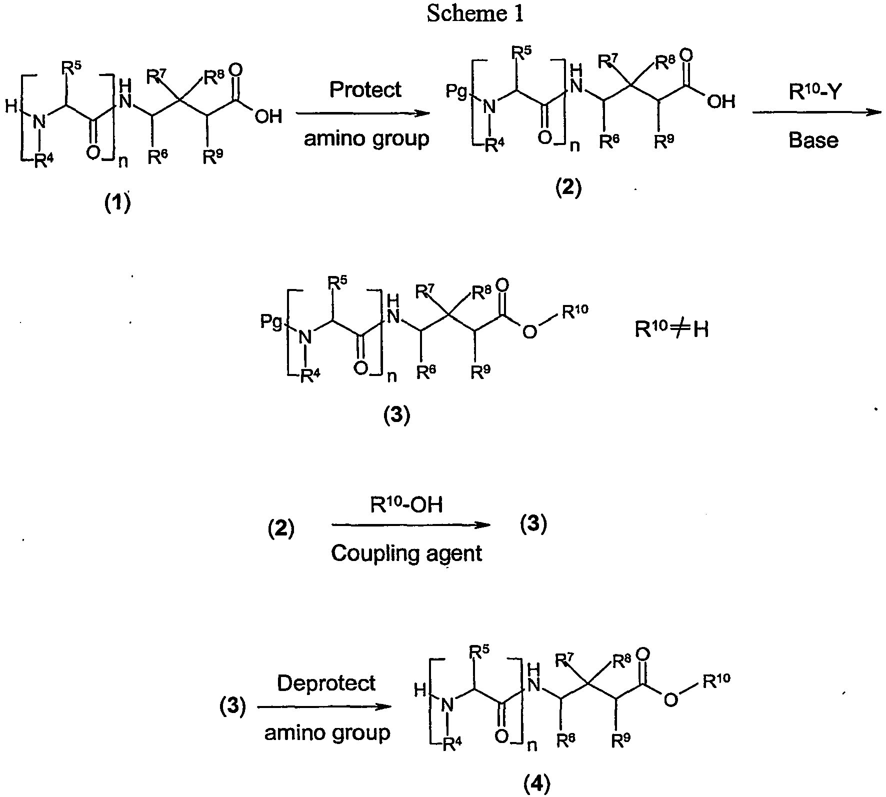

The amino group of (**1**) (a GABA analog when n = 0, or an aminoacyl derivative of a GABA analog when n =1) is protected under standard conditions with a protecting group ("Pg") to afford compound (**2**). The carboxylic acid moiety in (**2**) is esterified to yield compound (**3**), either (i) *via* alkylation with R¹⁰-Y, where Y is halide, O₃SR' (R' is alkyl, substituted alkyl, aryl or substituted aryl), or other suitable leaving group), or (ii) *via* condensation with alcohol R¹⁰-OH under standard acylation conditions (*e.g*., in the presence of a coupling agent such as a carbodiimide, *via* an acyl halide, acid anhydride or other activated ester intermediate). Removal of the protecting group from (3) under standard deprotection conditions affords compound (**4**). Preferably, when n = 0, the protecting group Pg is removable under acidic conditions and compound (**4**) is isolated as a salt, which is stabilized towards lactam formation relative to the corresponding free base form*. tert*-Butoxycarbonyl (*i.e*., Boc) is one preferred protecting group, and may be removed with HCl to afford (**4**) as a hydrochloride salt.

In a preferred embodiment, where n is 0, the hydrochloride salt of (**4**) is prepared directly from (1) by treatment with an excess of thionyl chloride or hydrogen chloride gas and alcohol R¹⁰-OH (Scheme 2). Typical ratios of (1) to thionyl chloride from between 1:1 and 1:20, and ratios of (1) to alcohol from between 1:1 and 1:20 may be used. The reaction may be performed at temperatures between-20 °C and 25°C. Under conditions where the alcohol R¹⁰-OH is a liquid, the alcohol may be used as a solvent for the reaction. Alternatively, the reaction may be performed in the presence of a suitable solvent, such as, for example, dichloromethane, dichloroethane, chloroform, toluene, dimethylformamide, dimethylacetamide, N-methylpyrrolidinone, or pyridine. Preferred alcohols R¹⁰-OH for this reaction are arylalkyl, substituted arylalkyl and allylic alcohols. Allyl alcohol and benzyl alcohol are particularly preferred.

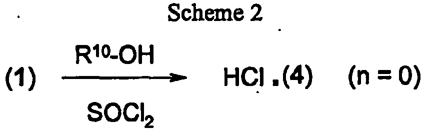

In one embodiment, a compound of formula (II) is prepared by acylation of (4) with compound (5) (see Scheme 3), where X is halide and Z is a leaving group (*e.g*., halide, *p*-nitrophenolate, imidazolyl, *etc.*). Preferably, X is Cl or Br and Z is Cl. More preferably, X and Z are both Cl. The acylation reaction may be performed in the presence of a base, including inorganic and organic bases (*e.g.*, tertiary amine bases, such as triethylamine, tributylamine, diisopropylethylamine, dimethylisopropylamine, N-methylmorpholine, N-methylpyrrolidine, N-methylpiperidine, pyridine, 2-methylpyridine, 2,6-dimethylpyridine, 4-dimethylaminopyridine, 1, 4-diazabicyclo[2.2.2]octane, 1, 8-diazabicyclo[5.4.0]undec-7-ene, 1, 5-diazabicyclo[4.3.0]undec-7-ene, *etc*.). Suitable solvents for this acylation include, but are not limited to, dichloromethane, dichloroethane, chloroform, toluene, dimethylformamide, dimethylacetamide, N-methylpyrrolidinone, dimethyl sulfoxide, pyridine, ethyl acetate, isopropyl acetate, acetonitrile, acetone, 2-butanone, methyl *tert*-butyl ether, or combinations thereof. Alternatively, biphasic solvent mixtures comprising water and one or more of dichloromethane, dichloroethane, chloroform, toluene, ethyl acetate, isopropyl acetate or methyl *tert*-butyl ether, may be utilized. Typical temperatures for performing this reaction are between-20 °C and 50°C, more preferably, between -20 °C and 25 °C.

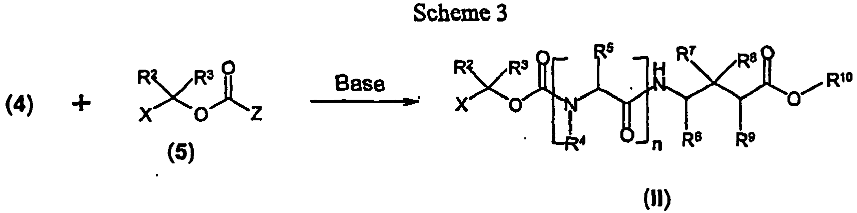

In another embodiment, a compound of formula (**II**), where R¹⁰ is trialkylsilyl or aryldialkylsilyl, may be prepared directly from compound (1) by silylation (*e.g*., using a silyl halide or silylamide reagent) and then acylation of the resulting intermediate with compound (5) (see Scheme 4). Suitable solvents for performing this reaction include, but are not limited to, dichloromethane, dichloroethane, chloroform, toluene, pyridine, and acetonitrile. Suitable bases for performing this reaction include, but are not limited to, triethylamine, tributylamine, diisopropylethylamine, dimethylisopropylamine, N-methylmorpholine, N-methylpynoliding, N-methylpiperidine, pyridine, 2-methylpyridine, 2,6-dimethylpyridine, 4-dimethylaminopyridine, 1, 4-diazabicyclo[2.2.2]octane, 1, 8-diazabicyclo[5.4.0]undec-7-ene or 1, 5-diazabicyclo[4.3.0]undec-7-ene. Typical temperatures for performing this reaction are between-78 °C and 50°C, more preferably, between 20 °C and 25°C.

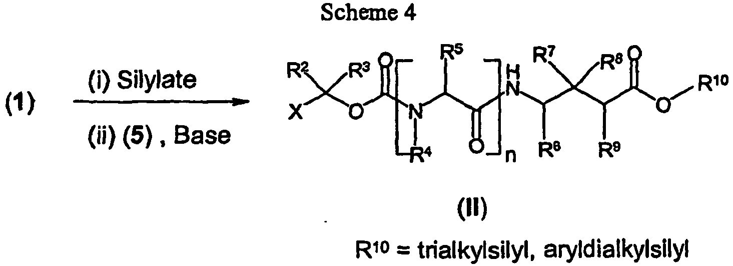

1-Acyloxylalkyl carbamates of formula (**I**) are prepared from compounds of formula (**II**) by treatment with carboxylic acids of formula (**III**) in the presence of an organic or inorganic base, or other metal salt, as illustrated in Scheme 5. Preferred solvents, bases and other reaction conditions have been described in detail previously (see Section 4.2 above).

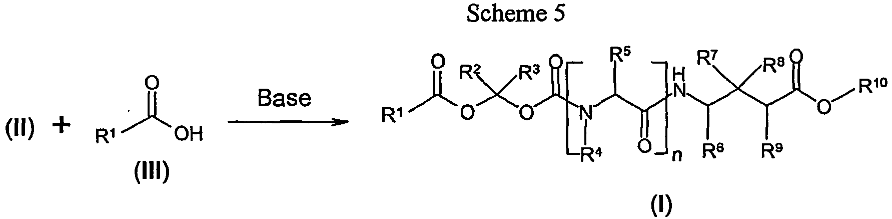

In one embodiment, R¹⁰ is a carboxylic acid protecting group that can be removed under mild conditions to provide a compound of formula (**I**) where R¹⁰ is hydrogen. Carboxylic acid protecting groups removable *via* mild acidic hydrolysis, fluoride ion-promoted hydrolysis, catalytic hydrogenolysis, transfer hydrogenolysis, or other transition metal-mediated deprotection reactions are preferred. In one embodiment, R¹⁰ is trimethylsilyl, allyl or benzyl.

### Examples

The invention is further defined by reference to the following examples.

In the examples below, the following abbreviations have the following meanings. If an abbreviation is not defined, it has its generally accepted meaning.

| | | |
|---|---|---|
| g | = | gram |
| h | = | hour |
| L | = | liter |
| LC/MS | = | liquid chromatography/mass spectroscopy |
| M | = | molar |
| min | = | minute |
| mL | = | milliliter |
| mmol | = | millimoles |

### Synthesis 1: Benzyl 1-Aminomethyl-1-Cyclohexane Acetate Hydrochloride (6)

A dry 500 mL, three-necked, round-bottomed flask was fitted with a magnetic stirring bar and a 60 mL pressure-equalizing addition funnel and flushed with nitrogen gas. The flask was charged with gabapentin (17.1 g, 0.1 mol) and benzyl alcohol (128 mL, 1.18 mol) and the mixture was cooled to 0°C with an ice-water bath. Thionyl chloride (51.8 mL, 77.25 g, 0.65 mol) was added dropwise to the stirred solution over a period of 1 h. The reaction was monitored by LC/MS, with product and unreacted gabapentin hydrochloride being observed. After stirring at room temperature for 3.5 days the reaction mixture contained residual gabapentin hydrochloride (by LC/MS). Additional thionyl chloride (20 mL, 30 g, 0.25 mol) was added at 0°C, and the reaction mixture allowed to stir at room temperature for another 12 h (LC/MS shows traces of residual gabapentin hydrochloride). A final portion of thionyl chloride (10 mL, 15 g, 0.12 mol) was added at 0°C and the reaction mixture allowed to stir at room temperature for 4 h (LC/MS showed no remaining gabapentin hydrochloride). The reaction mixture was then diluted with ethyl ether (200 mL) and cooled to 0°C while stirring. White crystalline solid formed, which was collected by filtration. The crude compound was recrystallized from a mixture of ethanol and ethyl ether (50 mL: 150 mL). Finally the white crystals were washed with 250 mL of ethyl acetate to give product (6) as a white solid (27 g, 91% yield). ¹H NMR (CDCl₃, 400 MHz.): δ 1.43 -1.52 (m, 10H), 2.64 (s, 2H), 3.08 (d, 2 H), 6.04 (br. s, 1H), 7.25 - 7.33 (m, 5H), 8.44 (br. s, 3H). MS (ESI) *m*/*z* 262.26 (M+H⁺).

### Synthesis 2: 1-[(tert-Butoxycarbonyl)aminomethyl]-1-Cyclohexane Acetic Acid (7)

Gabapentin, (700 g , 4.09 mol) was slurried in water (2.7 L) with potassium carbonate (1.2 kg, 8.58 mol) and mechanically stirred under a nitrogen atmosphere. Di-tert-butyl dicarbonate (875 g, 4.00 mol) was dissolved in dioxane (4 L) and was added in large aliquots while maintaining the pH at 8 - 10, and if needed, adjusting the pH using additional potassium carbonate. The reaction was monitored by ¹H-NMR, noting the disappearance of the singlet resonance at 1.22 ppm for di-*tert*-butyl dicarbonate. After stirring overnight at ambient temperature the dioxane was removed *in vacuo* and the pH of the aqueous phase adjusted to between 3 and 4 using 10% sulfuric acid. The aqueous mixture was extracted with ethyl acetate, the organic layer was washed with brine, dried over sodium sulfate and concentrated *in vacuo* to afford compound (7) as a white powder (893 g, 80% yield). ¹H NMR (CDCl₃), 400 MHz): δ 1.24 - 1.50 (m, 10H), 2.30 (s, 2H), 3.15 (d, 2H), 5.02 (t, 1H). MS (ESI) *m*/*z* 294.18 (M+Na⁺). Melting point: 125 -130 °C.

### Synthesis 3: Benzyl 1-[(tert-Butoxycarbonyl)aminomethyl]-1-Cyclohexane Acetate (8)

In a 12 L, multi-neck, round bottom flask with a bottom valve, mechanical stirrer and nitrogen blanket, (7) (1098 g, 4.05 mol) and potassium carbonate (838 g, 6.075 mol) were added to N-methylpyrrolidinone (2 L) at room temperature. Benzyl bromide (457 mL, 3.84 mol) was added over one hour. The strongly exothermic reaction was maintained below 40 °C, and the resulting white suspension was stirred until judged complete by NMR, about 2 hours.

Ice water (6 L) was carefully added to quench the reaction. Dichloromethane (3 L) was added, the organic phase separated and further extracted with water (2 x 2 L), 10% potassium carbonate (2 x 2 L), brine (2 L), then dried over sodium sulfate and concentrated *in vacuo.* The crude product (8) (∼1380 g, 94% yield) was carried to the next reaction without further purification. ¹H NMR (CDCl₃, 400 MHz): 1.33 - 1.53 (m, 10H), 2.32 (s, 2H), 3.11 (d, 2H), 5.01 (br. t, 1H), 5.09 (s, 2H), 7.31 - 7.35 (m, 5H). MS (ESI) *m*/*z* 442.39 (M+Na⁺).

### Synthesis 4: Benzyl 1-Aminomethyl-1-Cyclohexane Acetate Hydrochloride (6)

Compound (8) from Example 3 above was added slowly with stirring and cooling to a 5 L, 3-neck, round bottom flask containing 4N HCl in dioxane (2.5 L) over about a one hour period, the reaction mixture being maintained between 30 and 35 °C to keep the product from precipitating. The clear solution was divided into four aliquots, to each of which was added methyl-t-butyl ether (500 mL) to initiate crystallization of the product. Each batch fully solidified within 10 min. The solids were filtered, washed twice with ethyl acetate, then dried in a vacuum oven at 35 °C for 16 h to afford the product (6) as a white solid (936 g, 79% yield from (7)). ¹H NMR (CDCl₃, 400 MHz.): δ 1.43 - 1.52 (m, 10H), 2.64 (s, 2H), 3.08 (d, 2 H), 6.04 (br. s, 1H), 7.25 - 7.33 (m, 5H), 8.44 (br. s, 3H). MS (ESI) m/z 262.26 (M+H⁺).

### Synthesis 5: Benzyl 1-{[(α-Chloroethoxy)carbonyl]aminomethyl}-1-Cyclohexane Acetate (9)

In a 5-liter, 3-neck, round bottom flask, stirred mechanically and under nitrogen, was added dichloromethane (1.5 L), compound (**6**) (1.85 mol) and 1-chloroethyl chloroformate (258 g, 1.81 mol). The resulting solution was cooled to 15 °C and N-methylmorpholine (396 mL, 3.60 mol) was added slowly, with cooling, over a one hour period. The resulting turbid solution was stirred for 30 min, after which ¹H-NMR analysis showed the reaction to be complete. The reaction mixture was washed with water (2 x 2 L) and brine (1 L) and dried over sodium sulfate. Evaporation of the solvent afforded the title compound (9) as an orange oil (670 g, 98% yield). ¹HNMR (CDCl₃, 400MHz): 1.33 - 1.53 (m, 10H), 1.75 (d, 3H), 2.33 (s, 2H), 3.18 (d, 2 H), 5.09 (s, 2H), 5.58 (t, 1H), 6.53 (q, 1 H), 7.29 - 7.33 (m, 5H).

### Synthesis 6: Benzyl1-{[(α-Isobutanoyloxvethoxyl)carbonyl] aminomethyl}-1-Cyclobexane Acetate (10)

To a 10-L reactor equipped with a mechanical stirrer, cooling jackets and under nitrogen, was added isobutyric acid (1.35 L, 14.6 mol) *via* an addition funnel, followed by N-methyhnorpholine (1.6 L, 14.6 mol). The resulting solution was cooled to 16 °C and a solution containing compound (**9**) (1237 g crude, 2.93 mol) dissolved in isobutyric acid (1.35 L 14.6 mol) was slowly added with stirring. After the addition was complete the resulting turbid solution was stirred for a total of 33 h, after which time ¹H-NMR indicated less than 2% of starting material (**9**) remained. The crude reaction mixture was split in two equal batches, each of which was diluted with diethyl ether (6 L), washed with water (6 x 2L) in a centrifugal extractor to remove excess isobutyric acid, followed by washing with 10% potassium bicarbonate (4 x 2 L) and brine (2 x 2 L) before drying over anhydrous sodium sulfate. The combined organic extracts were concentrated to provide a dark orange oil (916 g). The crude oil (400 g) was loaded onto an 800 g Biotage™ silica gel chromatography column and eluted with 5% ethyl acetate in hexane (6 L), then with 7% ethyl acetate in hexane (12 L). The desired product elutes in the 7% fractions. The chromatographic purification was repeated with the remaining crude product to afford compound (10) as a thick colorless oil (822 g). ¹H NMR (CDCl₃, 400 MHz): δ 1.5 (d, 6H), 1.24 - 1.53 (m, 13 H), 1.45 (d, 3H), 2.33 (s, 2H), 2.48 - 2.55 (m, 1H), 3.16 (d, 2H), 5.09 (s, 2H), 5.35 (t, 1H), 6.77 (q, 1H), 7.29 - 7.36 (m, 5H). MS (ESI) *m*/*z* 442.48 (M+Na⁺):

### Synthesis 7: 1-{[(α-Isobutanoyloxyethoxy)carbonyl]aminomethyl}-1-Cyclohexane Acetic Acid (11)

Compound (**10**) (113 g) was dissolved in ethyl acetate (700 mL) and 10 g of 10% Pd-C was added. The reaction mixture was subjected to 50 psi of hydrogen gas in a Parr reactor for 40 min. Filtration through a sintered glass funnel and a hydrophobic membrane filtration cartridge (Millipore Opticap) removed the catalyst. The supernatant was concentrated to afford the product (**11**) as a white, crystalline solid (78 g, quantitative yield). Crystals formed in the freezer, then at room temperature over several days. ¹H NMR (CDCl₃, 400MHz): 1.15 (d, 6H), 1.40 - 1.55 (m, 10H), 1.45 (d, 3H), 2.32 (s, 2H), 2.49 - 2.56 (m, 1H), 3.23 (d, 2H), 5.41 (t, 1H), 6.75 (q, 1H). MS(ESI) *m*/*z* 330.29 (M+H⁺).

A portion of the product (25 g) was recrystallized by dissolution in 1 : 10 ethyl acetate : heptane (125 mL) at 60 °C, then slow cooling to 4°C. The white crystalline product (21 g) was isolated by filtration. Melting point: 63-64°C. Differential scanning calorimetry endotherm: 63 °C.

### Synthesis 8: Sodium 1-{[(α-Isobutanoyloxyethoxy)carbonyl]-aminomethyl}-1-Cyclohexane Acetate (12)

Compound (11) (540 g, 1.64 mol) was dissolved in acetone (850 mL) and water (500 mL) in a 4 L beaker, equipped with overhead stirring, pH meter and addition funnel. Aqueous sodium carbonate (1.0 M) was added slowly in 50 mL aliquots. After addition of 0.49 eq. base (803 mL of sodium carbonate solution) the pH was 7.3. Acetone was removed *in vacuo* at 25 °C and the pH re-checked to be ∼6.5. The remaining aqueous solution was divided into three 3-L flasks, shell frozen and lyophilized for two days. The resulting sodium salt (12) was scraped from the flasks as a hygroscopic solid and transferred quickly into bottles, which were immediately capped and transferred into a drying chamber at 14% relative humidity (RH). The remaining oily product in the flasks was dissolved in diethyl ether and concentrated *in vacuo* at 25 °C, then dried under high vacuum until a dry foam was produced.

### Synthesis 9: Allyl 1-Aminomethyl-1-Cyclohexane Acetate Hydrochloride (13)

A dry 500 mL, three-neck, round-bottomed flask was fitted with a magnetic stirring bar and a 100 mL pressure-equalizing addition funnel and flushed with nitrogen gas. The flask was charged with gabapentin (17.1 g, 0.1 mol) and allyl alcohol (100 mL, 1.46 mol) and the entire mixture was cooled to 0°C in an ice-water bath. Thionyl chloride (22.5 mL, 36 g, 0.3 mol) was added drop-wise over a period of 30 min to the stirred solution, and the reaction mixture allowed to stir for 16 h at room temperature. The mixture was then diluted with diethyl ether (200 mL) and cooled to 0°C while stirring. After several minutes white crystals formed, and were collected by filtration. The crude compound was recrystallized from a mixture of ethanol and diethyl ether (50 mL: 150 mL) to give the product (13) as a white solid (22 g, 88% yield), m.p: 138-142°C. ¹H NMR (CD₃OD, 400 MHz): δ 1.36-1.54 (m, 10H), 2:57 (s, 2H), 3.05 (s, 2H), 4.61 (d, 2H), 5.22 (dd, 1H), 5.33 (dd, 1H), 5.90-6.00 (m, 1H). MS (ESI) *m*/*z* 212.0 (M+H⁺).

### Synthesis 10: Allyl 1-{[(α-Chloroethoxy)carbonyl]aminomethyl}-1-Cyclohexane Acetate (14)

To a solution of compound (13) (30 g, 0.121 mol) in dichloromethane (100 mL) was slowly added 1-chloroethyl chloroformate (13 mL, 16.9 g, 0.119 mol). The reaction mixture was cooled to 0°C and N-methylmorpholine (26.39 mL, 24.28 g, 0.24 mol) was slowly added over a period of 1 h while maintaining a temperature of less than 10°C. The resulting turbid solution was stirred for 30 min, and the reaction mixture was then diluted with diethyl ether (250 mL), washed with water (100 mL) and brine (100 mL). The organic phase was dried over anhydrous sodium sulfate and concentrated to give the desired product (14) as light yellow viscous liquid (38 g, 99% yield). ¹H NMR (CDCl₃, 400 MHz): δ 1.35-1.58 (m, 10H), 1.78 (d, 3H), 2.32 (s, 2H), 3.22 (d, 2H), 4.57 (d, 2H), 5.25 (dd, 1H), 5.32 (dd, 1H), 5.52 (br. s, 1H), 5.90-5.94 (m, 1H), 6.54 (q, 1H).

### Synthesis 11: Allyl 1-{[(α-Isobutanoyloxyethoxy)carbonyl]aminomethyl}-1-Cyclohexane Acetate (15)

A solution of compound (**14**) (38 g, 0.12 mol) in isobutyric acid (55 mL, 52.5 g, 0.6 mol) was added to a mixture of isobutyric acid (55 mL, 52.5 g, 0.6 mol) in N-methylmorpholine (65 mL, 60 g, 0.59 mol) at 0°C over a period of 30 min. The resulting turbid solution was stirred for 16 h at room temperature. The reaction mixture was diluted with diethyl ether (500 mL) and washed with water (3 x 200 mL) followed by 10% potassium bicarbonate (4 x 200 mL) and brine (200 mL). The organic phase was dried over anhydrous sodium sulfate and concentrated to yield a viscous liquid. This crude product was purified by column chromatography on silica gel, eluting with 7.5% ethyl acetate : hexane to give the desired compound (15) as a clear, viscous liquid (37 g, 84% yield). ¹H NMR (CDCl₃, 400 MHz): δ 1.15 (d, 6H), 1.35-1.58 (m, 10H), 2.31 (s, 2H), 2.51 (m, 1H), 3.19 (d, 2H), 4.56 (d, 2H), 5.24 (dd, 1H), 5.32 (dd, 1H), 5.35 (br. s, 1H), 5.84-5.94 (m, 1H), 6.78 (q, 1H). MS (ESI) *m*/*z* 392.24 (M+H⁺).

### Synthesis 12: 1-{[(α-Isobutanoyloxyethoxy)carbonyl]aminomethyl}-1-Cyclohexane Acetic Acid (11)

***Method A:*** To a stirred suspension of ammonium formate (3.4 g, 54 mmol) in ethanol (34 mL), was added compound (**15**) (10 g, 27 mmol) together with 10% Pd/C (1g) under a nitrogen atmosphere. After one hour, the reaction mixture was filtered and the catalyst washed with ethanol (2 x 10 mL). The filtrates were combined and evaporated. The crude product was dissolved in diethyl ether (150 mL) and the organic phase was washed with 2N HCl (100 mL), water (100 mL) and brine (100 mL). The ether layer was dried over anhydrous sodium sulfate and concentrated to give a viscous liquid that crystallized upon standing. The product was recrystallized using 1:10 ethyl acetate : heptane (100 mL) to give the product (**11**) as a white, crystalline solid (7.9 g, 88%), m.p 63-64°C.

***Method B:*** To a stirred solution of compound (**15**) (1 g, 2.7 mmol) in acetonitrile (10 mL) under nitrogen was added (10 mg, 0.008 mmol) of tetrakis(triphenylphosphine) palladium (0) followed by morpholine (0.28 mL, 0.28 g, 3.2 mmol). After one hour, the solvent was removed *in vacuo.* The resulting oil was dissolved in diethyl ether (50 mL) and the organic phase was washed with 2N HCl (20 mL), water (20 mL) and brine (20 mL). The ether layer was dried over anhydrous sodium sulfate and concentrated to give an oil, which was purified by column chromatography on silica gel, eluting with 30% ethyl acetate : hexane. The desired product (**11**) was isolated as a white crystalline solid (0.75 g, 84% yield), m.p 63-64°C.

***Method C:*** To a stirred solution of compound (**15**) (1 g, 2.7 mmol) in dioxane (9 mL) was added ammonium formate (341 mg, 2.7 mmol) and palladium(II) acetate (12 mg) under a nitrogen atmosphere. The reaction mixture was heated to reflux for one hour and then concentrated *in vacuo.* The resulting oily residue was taken up in diethyl ether (50 mL), washed with 2N HCl (20 mL), water (20 mL) and brine (20 mL). The organic phase was dried over anhydrous sodium sulfate and evaporated to dryness. The crude compound was purified by column chromatography on silica gel, eluting with 30% ethyl acetate : hexane to give the desired product (**11**) as a colorless oil, which solidified on further standing at room temperature for 12 h (0.70 g, 78% yield), m.p. 62-64°C.

### Synthesis 13: 1-{[(α-Chloroethoxy)carbonyl]aminomethyl}-1-Cyclohexane Acetic Acid (16) via Trimethylsilyl 1-{[(α-Chloroethoxy)carbonyl]aminomethyl}-1-Cyclohexane Acetate (17)

In a 5-liter, 3-neck, round bottom flask containing dichloromethane (1.6 L) was added gabapentin (120.4 g, 0.704 mol) followed by triethylamine (294 mL, 2.11 mol). Chlorotrimethylsilane (178 mL, 1.40 mol) was slowly added while maintaining the reaction temperature below 15°C and the resulting suspension was stirred for 30 min. 1-Chloroethyl chloroformate (100 g, 0.704 mol) was then added slowly while maintaining the temperature below 15°C. After the addition was complete, additional triethylamine (88 mL, 0.63 mol) was added and the resulting suspension was stirred at room temperature for 30 min. The resulting silyl ester (**17**) was converted via acidic work-up to the corresponding acid (**16**) by washing the reaction mixture with water (2 x 1 L), followed by 1N HCl (2 x 2 L) then brine (2 x 500 mL). After drying over anhydrous sodium sulfate and removal of the solvent *in vacuo,* the crude product (190 g) was obtained as an orange oil and used in Example 14 without further purification. ¹H NMR (CDCl₃, 400 MHz): δ 1.41-1.57 (m, 10H), 1.78 (d, 3H), 2.33 (s, 2H), 3.27 (d, 2H), 5.42 (br. s, 1H), 6.55 (q, 1H).

### Synthesis 14: 1-{[(α-Isobutanoyloxyethoxy)carbonyl]aminomethyl}-1-Cyclohexane Acetic Acid (11)

To a 3-liter, 3-neck, round bottom flask was added isobutyric acid (254 g, 2.9 mol) followed by triethylamine (395 ml, 2.84 mol). The reaction mixture was cooled to room temperature and a solution of crude (**16**) from the above example (190 g, 0.69 mol) in dichloromethane (80 mL) was added in a controlled fashion to maintain the temperature below 30°C. The resulting pale yellow solution was stirred overnight. The reaction mixture was then diluted with one volume of dichloromethane and washed with water (6 x 500 mL), aqueous potassium bicarbonate (3 x 500 mL), and brine (2 x 500 mL). After drying over anhydrous sodium sulfate, removal of the solvent *in vacuo* afforded the crude product as a dark red oil (87 g). A portion (35 g) of this product was loaded onto an 800 g Biotage™ normal phase silica gel flash column and eluted with 40% diethyl ether in hexane (6 L), which after removal of the solvent *in vacuo* afforded product (11) as a colorless oil. This was repeated with a second 35 g portion of crude product yielding a further 13.5 g of (11). A sample of the product (25 g) was recrystallized by dissolution in heptane (325 mL) at 70 °C, then slow cooling to room temperature. The white crystalline product (11) (23 g) was isolated by filtration. Melting point: 63-64°C.

## Claims

1. Crystalline 1-{[(α-isobutanoyloxyethoxy)carbonyl]aminomethyl}-1-cyclohexane acetic acid having a melting point of 63-64°C or an endotherm of 63°C as measured by differential scanning calorimetry.

2. Crystalline 1-{[(α-isobutanoyloxyethoxy)carbonyl]aminomethyl}-1-cyclohexane acetic acid according to claim 1, having a melting point of 63-64°C.

3. Crystalline 1-{[(α-isobutanoyloxyethoxy)carbonyl]aminomethyl}-1-cyclohexane acetic acid according to claim 1, having an endotherm of 63°C as measured by differential scanning calorimetry.

4. Crystalline 1-{[(α-isobutanoyloxyethoxy)carbonyl]aminomethyl}-1-cyclohexane acetic acid according to claim 2, **obtainable by** recrystallization of 1-{[(α-isobutanoyloxyethoxy)carbonyl]aminomethyl}-1-cyclohexane acetic acid using 1 : 10 ethyl acetate : heptane.

5. Crystalline 1-{[(α-isobutanoyloxyethoxy)carbonyl]aminomethyl}-1-cyclohexane acetic acid according to claim 3, **obtainable by** recrystallization of 1-{[(α-isobutanoyloxyethoxy)carbonyl]aminomethyl}-1-cyclohexane acetic acid using 1 : 10 ethyl acetate : heptane.

6. Crystalline 1-{[(α-isobutanoyloxyethoxy)carbonyl]aminomethyl}-1-cyclohexane acetic acid according to claim 2, **obtainable by** recrystallization of -{[(α-isobutanoyloxyethoxy)carbonyl]aminomethyl}-1-cyclohexane acetic acid by dissolution in 1 : 10 ethyl acetate : heptane at 60°C, then slow cooling to 4°C.

7. Crystalline 1-{[(α-isobutanoyloxyethoxy)carbonyl]aminomethyl}-1-cyclohexane acetic acid according to claim 3, **obtainable by** recrystallization of 1-{[(α-isobutanoyloxyethoxy)carbonyl]aminomethyl}-1-cyclohexane acetic acid by dissolution in 1 : 10 ethyl acetate : heptane at 60°C, then slow cooling to 4°C.

8. Crystalline 1-{[(α-isobutanoyloxyethoxy)carbonyl]aminomethyl}-1-cyclohexane acetic acid according to claim 2, **obtainable by** recrystallization of 1-{[(α-isobutanoyloxyethoxy)carbonyl]aminomethyl}-1-cyclohexane acetic acid by dissolution in heptane at 70°C, then slow cooling to room temperature.

## Patentansprüche

1. Kristalline 1-{[(α-Isobutanoyloxyethoxy)carbonyl]aminomethyl}-1-cyclohexanessigsäure mit einem Schmelzpunkt von 63-64 °C oder einer mittels Differential-Scanning-Kalorimetrie gemessenen Endotherme von 63 °C.

2. Kristalline 1-{[(α-Isobutanoyloxyethoxy)carbonyl]aminomethyl}-1-cyclohexanessigsäure nach Anspruch 1 mit einem Schmelzpunkt von 63-64 °C.

3. Kristalline 1-{[(α-Isobutanoyloxyethoxy)carbonyl]aminomethyl}-1-cyclohexanessigsäure nach Anspruch 1 mit einer mittels Differential-Scanning-Kalorimetrie gemessenen Endotherme von 63 °C.

4. Kristalline 1-{[(α-Isobutanoyloxyethoxy)carbonyl]aminomethyl}-1-cyclohexanessigsäure nach Anspruch 2, die durch Umkristallisation von 1-{[(α-Isobutanoyloxyethoxy)carbonyl]aminomethyl}-1-cyclohexanessigsäure unter Verwendung von 1 : 10 Ethylacetat : Heptan erhältlich ist.

5. Kristalline 1-{[(α-Isobutanoyloxyethoxy)carbonyl]aminomethyl}-1-cyclohexanessigsäure nach Anspruch 3, die durch Umkristallisation von 1-{[(α-Isobutanoyloxyethoxy)carbonyl]aminomethyl}-1-cydohexanessigsäure unter Verwendung von 1 : 10 Ethylacetat : Heptan erhältlich ist.

6. Kristalline 1-{[(α-Isobutanoyloxyethoxy)carbonyl]aminomethyl}-1-cyclohexanessigsäure nach Anspruch 2, die durch Umkristallisation von 1-{[(α-Isobutanoyloxyethoxy)carbonyl]aminomethyl}-1-cyclohexanessigsäure mittels Lösen in 1 : 10 Ethylacetat : Heptan bei 60 °C und anschließendes langsames Abkühlen auf 4 °C erhältlich ist.

7. Kristalline 1-{[(α-Isobutanoyloxyethoxy)carbonyl]aminomethyl}-1-cyclohexanessigsäure nach Anspruch 3, die durch Umkristallisation von 1-{[(α-Isobutanoyloxyethoxy)carbonyl]aminomethyl}-1-cydohexanessigsäure mittels Lösen in 1 : 10 Ethylacetat : Heptan bei 60 °C und anschließendes langsames Abkühlen auf 4 °C erhältlich ist.

8. Kristalline 1-{[(a-Isobutanoyloxyethoxy)carbonyl]aminomethyl}-1-cyclohexanessigsäure nach Anspruch 2, die durch Umkristallisation von 1-{[(α-Isobutanoyloxyethoxy)carbonyl]aminomethyl}-1-cyclohexanessigsäure mittels Lösen in Heptan bei 70 °C und anschließendes langsames Abkühlen auf Raumtemperatur erhältlich ist.

## Revendications

1. Acide acétique 1-{[(α-isobutanoyloxyéthoxy)carbonyl]aminométhyle}-1-cyclohéxane cristallin ayant un point de fusion de 63-64 °C ou un endotherme de 63 °C comme mesuré par une analyse calorimétrique différentielle.

2. Acide acétique 1-{[(α-isobutanoyloxyéthoxy)carbonyl]aminométhyle}-1-cyclohéxane cristallin selon la revendication 1, ayant un point de fusion de 63-64 °C.

3. Acide acétique 1-{[(α-isobutanoyloxyéthoxy)carbonyl]aminométhyle}-1-cyclohéxane cristallin selon la revendication 1, ayant un endotherme de 63 °C comme mesuré par une analyse calorimétrique différentielle.

4. Acide acétique 1-{[(α-isobutanoyloxyéthoxy)carbonyl]aminométhyle}-1-cyclohéxane cristallin selon la revendication 2, pouvant être obtenu par le recristallisation de l'acide acétique 1-{[(α-isobutanoyloxyéthoxy)carbonyl]aminométhyle}-1-cydohéxane en utilisant 1 : 10 éthyle acétate : heptane.

5. Acide acétique 1-{[(α-isobutanoyloxyéthoxy)carbonyl]aminométhyl}-1-cyclohéxane cristallin selon la revendication 3, pouvant être obtenu par la recristallisation de l'acide acétique 1-{[(α-isobutanoyloxyéthoxy)carbonyl]aminométhyl}-1-cyclohéxane en utilisant 1 : 10 éthyle acétate : heptane.

6. Acide acétique 1-{[(a-isobutanoyloxyéthoxy)carbonyl]aminométhyl}-1-cyclohéxane cristallin selon la revendication 2, pouvant être obtenu par la recristallisation de l'acide acétique 1-{[(α-isobutanoyloxyéthoxy)carbonyl]aminométhyl}-1-cyclohéxane par dissolution dans 1 : 10 éthyle acétate : heptane à 60 °C, suivie d'un lent refroidissement à 4 °C.

7. Acide acétique 1 -{[(α-isobutanoyloxyéthoxy)carbonyl]aminométhyl}-1-cyclohéxane cristallin selon la revendication 3, pouvant être obtenu par la recristallisation de l'acide acétique 1-{[(α-isobutanoyloxyéthoxy)carbonyl]aminométhyl}-1-cyclohéxane par dissolution dans 1 : 10 éthyle acétate : heptane à 60 °C, suivie d'un lent refroidissement à 4 °C.

8. Acide acétique 1-{[(α-isobutanoyloxyéthoxy)carbonyl]aminométhyl}-1-cyclohéxane cristallin selon la revendication 2, pouvant être obtenu par la recristallisation de l'acide acétique 1 -{[(α-isobutanoyloxyéthoxy)carbonyl]aminométhyl}-1-cyclohéxane par dissolution dans de l'heptane à 70 °C, suivie d'un lent refroidissement à la température ambiante.
